# EUROPÄISCHE PATENTSCHRIFT

(11) **EP 1 778 609 B1**
(45) Veröffentlichungstag und Bekanntmachung des Hinweises auf die Patenterteilung: **20.06.2012**
(21) Anmeldenummer: 05787982.7
(22) Anmeldetag: 11.08.2005
(51) Int. Cl.: C07C 29/149, C07C 31/125

(54) **VERFAHREN ZUR HERSTELLUNG VON ALKOHOLEN**
METHOD FOR PRODUCING ALCOHOLS
PROCÉDÉ DE PRODUCTION D'ALCOOLS

(30) Priorität: 20.08.2004 FR 0451876
(43) Veröffentlichungstag der Anmeldung: 02.05.2007
(73) Patentinhaber: Cognis IP Management GmbH, 40589 Düsseldorf (DE); Cognis France, S.A.S., 77310 St. Fargeau Ponthierry (FR)
(72) Erfinder: TOPPHOFF, Magnus, 40625 Düsseldorf (DE); WÜRKERT, Stephan, 80639 München (DE); BIZ, Nicolas, F-45220 Gy les Nomains (FR); RIGAL, Jean, F-31170 Tournefeuille (FR); PELZER, Gerrit, Khet Klontoey, Bangkok 10110 (TH)
(86) Internationale Anmeldenummer: PCT/EP2005/008738
(87) Internationale Veröffentlichungsnummer: WO 2006/021328

(56) Entgegenhaltungen:
- EP-A- 0 548 920
- DE-B- 1 005 497
- GB-A- 1 512 751
- GB-A- 2 250 287
- US-A- 5 157 168
- DATABASE WPI Section Ch, Week 199718 Derwent Publications Ltd., London, GB; Class E17, AN 1997-197225 XP002362046 & JP 09 052853 A (KAO CORP) 25. Februar 1997 (1997-02-25)

## Beschreibung

### Gebiet der Erfindung

Die Erfindung betrifft ein Verfahren zur Herstellung von Alkoholen.

### Stand der Technik

Fettalkohole werden seit langem durch katalytische Hydrierung von Fettsäuren, Fettsäuremethylestern oder Fettsäuretriglyceriden hergestellt. Hierzu gibt es eine umfangreiche Literatur, die bereits in die 30er Jahre des letzten Jahrhunderts zurückreicht. Beispielhaft seien drei Übersichtsartikel genannt, denen wichtige Grundlagen des hier in Rede stehenden Fachgebietes entnommen werden können:
- J.W.E. Coenen (Fette, Seifen, Anstrichmittel 1975, 77(9), 341-347): "Katalytische Prozesse auf dem Gebiet industrieller Fettsäureproduktion I"
- W. Zschau (Fette, Seifen, Anstrichmittel 1979, 81(8), 303-310): "Aspekte der Hydrierung von Fetten und Fettsäuren"
- Theodor Voeste et al. (JAOCS 1984, 61 (2), 350-52): "Production of Fatty Alcohols from Fatty Acids"

In der großindustriellen Praxis der Fettalkoholherstellung sind nach wie vor Fettsäuremethylester die Rohstoffe der Wahl. Allerdings ist auch mehrfach die Hydrierung von Fettsäuretriglyceriden und die Hydrierung von Fettsäuren vorgeschlagen worden. Letztere ist technisch besonders schwer zu beherrschen, weil freie Fettsäuren in mehrfacher Hinsicht Probleme verursachen: Zum einen sind sie sehr korrosiv, was nicht nur für die Werkstoffe der Hydrieranlage eine Rolle spielt (hier ist bekanntermaßen Edelstahl erforderlich), sondern auch die Lebensdauer und Aktivität des eingesetzten Katalysators empfindlich mindern kann. Zum anderen ist die Carboxylgruppe eine außerordentlich schwer zu hydrierende Gruppe, was sich darin äußert, daß man unter forcierten Bedingungen zu arbeiten gezwungen ist - üblicherweise arbeitet man bei Temperaturen im Bereich von 150 bis 320 °C, Drucken im Bereich von 20 bis 300 bar und hohen Überschüssen an Wasserstoff -, was einerseits energieintensiv ist, andererseits die Bildung von Nebenprodukten begünstigt. Im Hinblick auf die eingesetzten Katalysatoren haben sich Cu-Chromit-Katalysatoren besonders bewährt und sind dem Fachmann als sogenannte Adkins-Katalysatoren seit langem bekannt.

Aus WO 93/00159 (Henkel) sind Schachtreaktoren für Feststoff-katalysierte Gas-Flüssigreaktionen bekannt, die zum Hydrieren von Fettsäuren bzw. Fettsäureestern insbesondere für die Rieselfahrweise dienen können, und die mindestens ein zumindest teilweise innerhalb des Katalysatorfestbetts angeordnetes Kühlelement enthält, mit dem eine gute Wärmeabfuhr erreicht werden soll.

Aus CH 169,232 (I.G.Farbenindustrie) ist bekannt, die Hydrierung von Fettsäuren in Gegenwart von Lösungs- oder Verdünnungsmitteln durchzuführen. Hierzu werden niedermolekulare aliphatische Alkohole vorgeschlagen.

Der Einsatz von Lösemitteln, insbesondere niedere aliphatische Alkohole mit 1 bis 4 Kohlenstoffatomen, ist auch für die Hydrierung von Methylestern di- und oligomerer Fettsäuren oder deren Estern vorgeschlagen worden, vergleiche hierzu die Lehre der DE 17,68,313 (Henkel).

Aus DE 670,832 (Deutsche Hydrierwerke AG) ist bekannt, die Hydrierung von Fettsäuren in Gegenwart von höhersiedenden Alkoholen durchzuführen. Dabei soll der Siedepunkt dieser Alkohole nicht mehr als 50 °C unter dem der eingesetzten Fettsäure liegen. Die Menge des Alkohols soll mindestens ausreichen, damit neutraler Ester gebildet werden kann.

Die genannte DE 670832 wirkt bereits wie ein Vorgriff auf die wesentlich später von Lurgi eingeführte Wachsester-Technolgie, die Mitte der 60er Jahre bekannt wurde, vergleiche US-A-3,180,898. Hierbei werden Fettsäuren mit den korrespondierenden Fettalkoholen zu Estern umgesetzt, den sogenannten Wachsestern, die in einem Suspensionsverfahren hydriert werden. Das hier angesprochene Lurgi-Eintopf-Slurry-Verfahren arbeitet mit dem Verdünnungsprinzip, insofern ein Verhältnis von Fettalkohol zu Fettsäure von mindestens 250 : 1 eingestellt wird. Später hat Lurgi ein verbessertes Verfahren zum Erzeugen von Wachsestern entwickelt, vergleiche DE 43,43,320).Darüber hinaus wurde diese Thematik in EP-B-737,664 publiziert.

Aus US 2,759,955 (Societe Belge) ist die katalysatorfreie Veresterung von Fettsäuren mit Methanol bekannt. Diese verläuft dann schnell und in hohen Ausbeuten, wenn man unter Bedingungen arbeitet, bei denen Methanol im überkritischen Zustand vorliegt. Die kritischen Bedingungen für Methanol lauten, daß die Temperatur mindestens 240 °C und der Druck mindestens etwa 80 bar betragen muß. Es werden hohe Umsetzungsgrade von bis zu 96% erzielt.

Eine Untergrenze für den Umsetzungsgrad ist der Druckschrift nicht unmittelbar als Zahlenwert zu entnehmen. Gemäß Tabelle I (vergleiche in Spalte 2, Zeilen 40-52 der US 2,759,955) beträgt der Umsetzungsgrad bei 200 °C 47% , bei 230 °C 54% und bei 260 °C 80%. Da die kritische Temperatur für Methanol bei 240 °C liegt, dürfte der Umsetzungsgrad zumindest in der Größenordnung von etwa 60% liegen.

Gebildetes Reaktionswasser wird während des Veresterungsschrittes selbst nicht entfernt. Die Aufarbeitung geschieht in der Weise, daß Restmethanol und (bei der Veresterung gebildetes) Wasser nach der Veresterung durch Flash-Evaporation abgetrennt werden und der Fettsäureester aus dem dabei erhaltenen rohen Gemisch von Fettsäureester und Fettsäure zwecks Reinigung abdestilliert wird.

In diesem Zusammenhang wird ausdrücklich offenbart, daß die nicht umgesetzte Fettsäure aus dem rohen Gemisch von Fettsäureester und Fettsäure nicht vom Fettsäuremethylester abgetrennt werden muß, sofern man anschließend zum Fettalkohol weiterhydrieren will.

Es wird also gelehrt, daß die nach dem Verfahren gemäß der US 2,759,955 nach Flash-Evaporation zugänglichen rohen Gemische von Fettsäureester und Fettsäure ohne weiteres der Hydrierung zugeführt werden können. Mithin offenbart die US 2,759,955 inter alia ein Verfahren zur Hydrierung von Mischungen von Fettsäuremethylestern und den korrespondierenden Fettsäuren zu Fettalkoholen, wobei in dieser Mischung der Anteil an Fettsäuremethylestern mengenmäßig überwiegt und wobei in diesen Mischungen kein Wasser und kein Methanol vorhanden ist.

Aus FR-A-2,818,275 (Cognis) ist ein Verfahren zur Direkt-Hydrierung von Carbonsäuren zu den korrespondierenden Alkoholen in Gegenwart von heterogenen Katalysatoren bekannt, wobei man Carbonsäuren in gelöster bzw. dispergierter Form einsetzt. Als Lösungs- bzw. Dispergiermedien dienen dabei niedere Alkohole und/oder Fettsäureniedrigalkylester. Dadurch soll einerseits die katalytische Aktivität und die Lebensdauer der eingesetzten Katalysatoren, beispielsweise Cu-Chromit, verbessert werden. In einer bevorzugten Ausführungsform werden als Dispergiermittel Gemische von Methanol und Fettsäuremethylestern, eingesetzt. Dies wird im Beispiel 2 der Anmeldung demonstriert, bei dem Palmkernfettsäure in einem Dispergiermittel (Mischung aus Methanol und Palmkernfettsäuremethylester im Molverhältnis 1 : 1) bei einem molaren Verhältnis von Palmkernfettsäure zu Dispergiermittel von 1 : 8 in Gegenwart eines handelsüblichen Cu-Chromitkatalysators hydriert wurde.

GB-A-2,250,287 (Unichema) lehrt, daß sich Cu-haltige Katalysatoren, insbesondere Kupfer-Zink-Katalysatoren, die im Rahmen der Hydrierung von Fettsäureestern zu Fettalkoholen eingesetzt werden, bei nachlassender Aktivität durch Wasserdampf regenerieren lassen. Die technische Lehre besteht mithin darin, daß Wasser sich zur Katalysator-Regeneration eignet und somit die Standzeiten von Katalysatoren verlängert. In einer Ausführungsform wird der Fettsäureester zusammen mit 0,1 bis 2% Wasser bei der Hydrierung eingesetzt. Dies veranschaulicht ein Experiment, das unter "Beispiel 1" geschildert wird: Hierbei wurde zunächst ein Fettsäuremethylester hydriert, wobei der Umsetzungsgrad 97,2% betrug. Nach einer Laufzeit von 50 Stunden war der Umsetzungsgrad auf 97,0% gefallen. An dieser Stelle wurde dem Rohstoff 1,2% Wasser zugesetzt. Danach stieg der Umsetzungsgrad auf 97,7% an.

Aus DE-B-10,05,497 (Dehydag) ist bekannt, daß bei der Hydrierung von Fettsäureestern mit niedermolekularerer Alkoholkomponente über stückigen Katalysatoren der Zusatz niedermolekularer Alkohole, z.B. Methanol, eine Steigerung des Durchsatzes erlaubt und außerdem die Lebensdauer des Katalysators und die Qualität des erhaltenen Fettalkohols verbessert. Letzteres äußert sich in einer Verminderung der Bildung von Kohlenwasserstoffen, die im Zuge der Hydrierung als unerwünschte Nebenprodukte entstehen.

DE-A-26,13,226 (Henkel) beschreibt bei der Würdigung des Standes der Technik, es sei bekannt, Fettalkohole durch katalytische Hochdruckhydrierung von Fettsäuren, deren Estern oder Anhydriden mit Wasserstoff oder wasserstoffhaltigen Gasen bei erhöhter Temperatur in der Weise vorzunehmen, daß man die Ausgangsstoffe gleichzeitig mit Wasserstoff oder den wasserstoffhaltigen Gasen im strömenden System bei Drucken im Bereich von 100 bis 500 atm über stückigen Katalysator, etwa Kupferchromit, leitet. Dabei kann zur Schonung des Kontaktes und zur Erzielung eines qualitativ hochwertigen Fettalkohols Methanol im Kreislaufgas der Hydrierung mitzuführen. Gemäß der Lehre der DE-A-2613226 läßt sich dieses Methanol vorteilhafterweise durch Inertgase wie beispielsweise Stickstoff ersetzen. Als Ausgangsstoffe werden Fettsäuren und/oder deren Ester eingesetzt. Der Ersatz des Methanols durch das Inertgas bedingt eine Steigerung des Durchsatzes, einen besseren Wärmeübergang des Kreisgases.

DE-A-40,37,731 offenbart spezielle Kupfer-Chromoxid-Katalysatoren, insbesondere für die Hydrierung von Fettsäuren und Fettsäureestern sowie die Verwendung dieser Katalysatoren zur Sumpfphasen-Hydrierung von Fettsäuren bzw. Fettsäuregemischen mit 5 bis 24 C-Atomen und/oder deren Estern, gegebenenfalls im Gemisch mit Alkoholen, in die entsprechenden Fettalkohole.

WO-A-94/06738 (Unichema) schlägt vor, daß sich Fettsäuren bzw. deren Ester in flüssiger Phase dadurch gut hydrieren lassen, wenn man in Gegenwart eines flüssigen inerten Wasserstofflrägers arbeitet. Bei diesem Wasserstoffträger handelt es sich um eine Substanz, die eine gute Löslichkeit für Wasserstoff aufweist. Dazu eignen sich aliphatische und aromatische Kohlenwasserstoffe (insbesondere werden Alkane wie Pentan und Octan erwähnt) und Wachsester. Durch die gute Löslichkeit von Wasserstoff in den genannten Substanzen lassen sich mildere Reaktionsbedingungen bei der Hydrierung einstellen, was konkret bedeutet, daß man bei vergleichsweise niederen Drucken im Bereich von 20 bis 100 bar arbeiten kann.

JP-A-H09-052853 (Kao) beschreibt ein Verfahren zur Herstellung von Fettalkoholen durch Hydrierung in Gegenwart eines stückigen Katalysators, wobei man als Ausgangsstoffe Gemische von Fettsäureestern und Fettsäuren einsetzt. Dabei beträgt der Gehalt dieser Gemische an Fettsäuren 0,1 bis 30 Gew.-%, vorzugsweise 0,1 bis 5 Gew.-%. Es wird angegeben, daß durch den genannten Anteil des binären Gemisches an Fettsäure weniger Kohlenwasserstoffe und Ether als Nebenprodukte gebildet werden.

### Beschreibung der Erfindung

Wie bereits erwähnt werden Fettalkohole üblicherweise ausgehend von nativen Ölen und Fetten, Fettsäuren bzw. Fettsäureestern durch Kontaktreduktion hergestellt. Die dabei auftretenden technischen Probleme sind komplexer Natur und reichen wie beschrieben vom Verlust der Katalysatoraktivität bis zur unerwünschten Nebenproduktbildung.

Aufgabe der vorliegenden Erfindung war es, ein - insbesondere kontinuierliches - Verfahren bereitzustellen, bei dem Carbonsäureester (insbesondere: Fettsäurealkylester) bzw. Mischungen von Carbonsäureestern und Carbonsäuren (insbesondere: Mischungen von Fettsäurealkylestern und Fettsäuren) in guten Ausbeuten und mit geringer Nebenproduktbildung zu den entsprechenden Alkoholen (insbesondere: Fettalkoholen) hydriert werden können.

Insbesondere sollte der Gehalt der bei der Hydrierung erhaltenen Alkohole an Kohlenwasserstoffen, sekundären Alkoholen, Iso-Alkoholen und Ethern so gering wie möglich sein.

Unter Kohlenwasserstoffen werden gesättigte Kohlenwasserstoffe verstanden. Diese verunreinigen nicht nur das Zielprodukt, also die bei der Hydrierung entstehenden Alkohole, sondern sie beeinträchtigen auf Dauer auch das Hydriersystem, weil sich Kohlenwasserstoffe im Kreisgas anreichern und den Partialdruck des darin enthaltenen Wasserstoffs vermindern, was wiederum zu schlechteren Hydrierergebnissen führt.

Die beiden Verunreinigungen sekundäre Alkohole und Iso-Alkohole sind insbesondere deswegen von Bedeutung, weil sie unerwünschte Isomere der angestrebten Zielprodukte sind. Setzt man beispielsweise bei der Hydrierung als Rohstoffe Fettsäuremethylester und/oder Fettsäuren ein, so sind die Zielprodukte der Hydrierung Fettalkohole. Hierbei handelt es sich um lineare Alkohole mit endständiger OH-Gruppe. Sekundäre Alkohole stellen daher als Isomere der Fettalkohole, die ja primäre Alkohole sind, unerwünschte Nebenprodukte dar. Analog sind Iso-Alkohole unerwünschte Nebenprodukte, da bei ihnen zwar (wie in den Fettalkoholen) eine terminale OH-Gruppe vorliegt, jedoch zusätzlich und im Gegensatz zu den Fettalkoholen mindestens eine Alkylverzweigung in der Kohlenstoffkette vorliegt. Ziel war es daher, ein Verfahren zur Herstellung von weitgehend isomerenreinen Fettalkohol-Zusammensetzungen bereitzustellen, bei dem die Bildung der Nebenprodukte sekundäre Alkohole und iso-Alkohole so weit wie möglich unterbunden ist

Der Eindeutigkeit halber sei festgestellt, daß im Rahmen der vorliegenden Erfindung unter Iso-Alkoholen Alkyl-verzweigte, insbesondere Methyl-verzweigte Alkohole und unter sekundären Alkoholen Alkohole mit mindestens einer sekundären OH-Gruppe verstanden werden. So ist beispielsweise 2-Methyl-undecan-1-ol ein Iso-Alkohol und Dodecan-2-ol ist ein sekundärer Alkohol.

Unter Ethern werden im Rahmen der vorliegenden Erfindung Dialkylether verstanden.

Eine weitere Aufgabe war es, dass der bei dem Verfahren einzusetzende stückige Katalysator (Festbettkatalysator) eine lange Lebensdauer ohne merkliche Einbußen der katalytischen Aktivität behalten sollte.

Das erfindungsgemäß bereitzustellende Verfahren mit den geschilderten Qualitätsverbesserungen sollte sich insbesondere zur Herstellung von Fettalkoholen eignen.

Überraschenderweise wurde nun gefunden, daß sich Carbonsäureester bzw. Mischungen von Carbonsäureestern und Carbonsäuren in guten Ausbeuten und mit erheblich verringerter Nebenproduktbildung - insbesondere mit deutlich verringerter Bildung an Kohlenwasserstoffen, Iso-Alkoholen, sekundären Alkoholen und Ethern - zu den entsprechenden Alkoholen hydrieren lassen, wenn man den Einsatzstoffen in bestimmten Mengen Wasser zusetzt.

Die erforderlichen Mengen an Wasser sind vergleichsweise hoch. Dies ist für den Fachmann besonders überraschend; denn soweit der Anmelderin bekannt ist, wird bei der Hydrierung von Fettstoffen, insbesondere von Fettsäuremethylestern oder Fettsäuren, mit Ausnahme der oben zitierten GB-A-2,250,287 ein Zusatz von Wasser zum zu hydrierenden Gut geradezu vermieden. Dies gilt vor allem bei der Hydrierung von Fettsäuren, wo z.B. Coenen (siehe hierzu den oben zitierten Review) ausdrücklich ausführt daß Wasser die Hydrierung verzögert. Ferner ist Wasser gerade in Verbindung mit Fettsäuren unerwünscht: a) weil ein Korrosionsproblem entsteht für das Reaktor-Equipment und b) weil dies die Lebensdauer des Katalysators verringert.

Insgesamt kann es als ein wichtiger Aspekt das Wissens des Fachmanns auf dem hier in Rede stehenden Gebiet (Hydrierung von Fettsäuren und/oder Fettsäurealkylestern zu Fettalkoholen) bezüglich der Qualität der Rohstoffe angesehen werden, daß die bei der Hydrierung einzusetzenden Rohstoffe möglichst frei von Wasser sein sollen. Die vorliegende Erfindung kann in diesem Sinne durchaus als Überwindung eines Vorurteils betrachtet werden.

Gegenstand der Erfindung ist ein Verfahren zur Herstellung von Fettalkoholen, indem man eine Mischung enthaltend
(a) 10 bis 70 Gew.-% ein oder mehrere C₈₋₂₄-Fettsäure-C₁₋₃-alkylester,
(b) 20 bis 80 Gew.-% ein oder mehrere C₈₋₂₄-Fettsäuren,
(c) 1 bis 65 Gew.-% ein oder mehrere C₁₋₃-Alkanole und
(d) 1,5 bis 20 Gew.-% Wasser,
wobei sich die Gew.-%-Angaben der Komponenten (a) bis (d) jeweils auf die Summe der Komponenten (a) bis (d) beziehen,
in Gegenwart eines heterogenen stückigen Katalysators kontinuierlich hydriert,
mit der zusätzlichen Maßgabe, daß
- die Summe der Anteile der Komponenten (a) bis (d) den Wert 100 Gew.-% ergibt und
- die Menge an Wasser (d) - bezogen auf die Menge an C₈₋₂₄-Fettsäure-C₁₋₃-alkylester (a) - mindestens 3,4 Gew.-% beträgt.

In einer Ausführungsform enthält die zu hydrierende Mischung:
(a) 10 bis 60 Gew.-% ein oder mehrere C₈₋₂₄-Fettsäuremethylester,
(b) 20 bis 60 Gew.-% ein oder mehrere C₈₋₂₄-Fettsäuren,
(c) 1 bis 60 Gew.-% Methanol und
(d) 2,0 bis 15 Gew.-% Wasser.

Vorzugsweise wird das zu hydrierende Gut, also die Mischung mit den Komponenten (a) bis (d) in kontinuierlicher Fahrweise hydriert, insbesondere in einem Rohr- oder Schachtreaktor, der mit stückigem Kupferchromit-Katalysator beschickt ist, wobei eine Rieselfahrweise bevorzugt ist. Wie dem Fachmann bekannt ist unter Rieselfahrweise zu verstehen, daß sowohl das zu hydrierende Gut als auch der Wasserstoff von oben in den lotrecht stehenden Rohr- oder Schachtreaktor eintritt und das Reaktionsprodukt den Reaktor unten verlässt.

### Quaternäre Systeme

alle vier Komponenten (a) bis (d). Die zu hydrierende Mischung enthält zwingend alle vier Komponenten (a) bis (d).

Vorzugsweise setzt man hierbei als Komponente (a) C₁₋₃-Alkylester von C₈₋₂₄-Fettsäuren ein und als Komponente (b) C₈₋₂₄-Fettsäuren, insbesondere dieselben, die in Komponente (a) als Bausteine vorhanden sind. Setzt man also beispielsweise als Komponente (a) den Methylester einer C_{12/14}-Fettsäure ein, so wählt man als Komponente (b) vorzugsweise ebenfalls eine C_{12/14}-Fettsäure, insbesondere eine mit derselben Rohstoffbasis.

Die gemäß dem erfindungsgemäßen Verfahren einzusetzenden Mischungen mit den Komponenten (a) bis (d) können an sich nach jeder dem Fachmann geläufigen Methode hergestellt werden.

In einer Ausführungsform stellt man die zu hydrierende quaternäre Mischung der Komponenten (a) bis (d) her, indem man die Komponenten miteinander mischt.

In einer anderen Ausführungsform stellt man die zu hydrierende quaternäre Mischung der Komponenten (a) bis (d) her, indem man ein oder mehrere Carbonsäuren (b) mit ein oder mehreren Alkoholen (c) mischt - wobei ein Gewichtsverhältnis im Bereich von 10 : 90 bis 90 : 10 bevorzugt ist - und bei erhöhtem Druck und erhöhter Temperatur in Abwesenheit eines Katalysators miteinander im Sinne einer zumindest partiellen Veresterung umsetzt. Unter erhöhtem Druck ist dabei zu verstehen, daß der Druck bei der Veresterung mindestens 60 bar, vorzugsweise mindestens 80 bar beträgt. Unter erhöhter Temperatur ist dabei zu verstehen, daß die Temperatur mindestens 200 °C, vorzugsweise mindestens 220 °C und insbesondere mindestens 240 °C beträgt. Die so erhaltenen quaternären Mischungen der Komponenten (a) bis (d) werden unmittelbar der Hydrierung zugeführt.

Die genannte zumindest partielle Veresterung der Carbonsäuren (b) mit den Alkoholen (c) - ganz besonders bevorzugt ist hierbei der Einsatz von C₈₋₂₄-Fettsäuren als Komponente (b) und von Methanol als Komponente (c) - ist dabei so weit zu führen, daß die dabei entstehende quaternäre Mischung die Komponenten (a) bis (d) in den oben genannten Mengen enthält.

In einer bevorzugten Ausführungsform wird dies dadurch erreicht, daß man die Komponenten (b) und (c), insbesondere C₈₋₂₄-Fettsäuren und Methanol, in einem Vorlagebehälter in flüssiger Form (dazu reichen üblicherweise moderate Temperaturen im Bereich von etwa 20 bis 70 °C) miteinander mischt und diese Mischung durch eine Vorrichtung führt, in der ein Druck von mindestens 80 bar und eine Temperatur von mindestens 200 °C, vorzugsweise mindestens 220 °C und insbesondere mindestens 240 °C herrscht. Bei dieser Vorrichtung kann es sich beispielsweise um ein Rohr oder um einen Wärmetauscher handeln. In diesem Zusammenhang (katalysatorfreie Veresterung) sei auf die eingangs genannte US 2,759,955 verwiesen. Die Dimensionierung der Vorrichtung (Rohr oder Wärmetauscher) kann vom Fachmann in weiten Bereichen variiert werden. Bei geeigneter Wahl von Rohrlänge und Rohrdurchmesser und gegebenenfalls Nutzung von Elementen, die im Rohr für turbulente Strömung sorgen, kann der Fachmann eine Optimierung derart vornehmen, daß bereits bei kurzen Reaktionszeiten hohe Veresterungsgrade erreicht werden, wodurch es ermöglicht wird, mit Durchflussgeschwindigkeiten im Bereich von 0,5 bis 10 Minuten auszukommen. Die auf diese beschriebene Weise erhältliche Mischung der Komponenten (a) bis (d) wird vorzugsweise in einen Schachtreaktor eingespeist, der mit insbesondere Kupferchromitpellets gefüllt ist.

In einer anderen Ausführungsform wird dies erreicht, indem man die Komponenten (b) und (c), insbesondere C₈₋₂₄-Fettsäuren und Methanol, in einem Vorlagebehälter bei moderaten Temperaturen (etwa 20 bis 70 °C) mischt und diese Mischung ohne sie weiter zu erwärmen in einen Schachtreaktor einspeist, in dessen unteren Teil sich die Aufschüttung des Hydrierkatalysators, insbesondere Pellets aus Kupferchromit, und in dessen oberen Teil sich eine inerte feste stückige Substanz, etwa Glaskugeln, befindet. Auf diese Weise dient der obere Teil des Hydrierreaktors der Herstellung der erfindungsgemäß bevorzugt einzusetzenden quaternären Mischung der Komponenten (a) bis (d) in den angegebenen Mengenverhältnissen. Im Zusammenhang mit dieser Ausführungsform kann man sich beispielsweise eines Schachtreaktors mit ein oder mehreren integrierten Wärmetauschern bedienen, wie er beispielsweise in WO 93/00159 beschrieben ist (dort werden die Wärmetauscher als Kühlelemente bezeichnet, weil sie gemäß der Lehre der Schrift dazu dienen, die Exothermie abzuführen).

In einer weiteren Ausführungsform stellt man die zu hydrierende quaternäre Mischung der Komponenten (a) bis (d) her, indem man die Komponenten (b) und (c), insbesondere C₈₋₂₄-Fettsäuren und Methanol, in einem Vorlagebehälter bei moderaten Temperaturen (etwa 20 bis 70 °C) mischt und diese Mischung - vorzugsweise bei erhöhtem Druck und erhöhter Temperatur - durch einen Veresterungsreaktor leitet, der einen festen, immobilisierten Veresterungskatalysator, etwa einen sauren Ionenaustauscher, enthält. Die erhaltene quaternäre Mischung wird anschließend in einen HydrierReaktor geleitet.

### Zu den Carbonsäuren (a)

In einer Ausführungsform handelt es sich bei den Verbindungen (a) um Alkylester von C₈₋₂₄-Fettsäuren, insbesondere um C₁₋₃-Alkylester von C₈₋₂₄-Fettsäuren.

In einer weiteren Ausführungsform handelt es sich bei den Verbindungen (a) um Alkylester von C₃₋₂₄-alpha-omega-Dicarbonsäuren, wobei C₁₋₃-Alkylester von C₃₋₁₂-alpha-omega-Dicarbonsäuren bevorzugt sind. Hierbei kann es sich um Vollester oder um Partialester handeln, wobei Vollester bevorzugt sind.

In einer weiteren Ausführungsform handelt es sich bei den Verbindungen (a) um Alkylester von Dimer- und/oder Trimerfettsäuren, insbesondere um C₁₋₃-Alkylester von Dimer- und/oder Trimerfettsäuren.

Alkylester von Carbonsäuren (a) - nachfolgend auch kurz Carbonsäurealkylester genannt - sind aus einem Carbonsäurebaustein und - je nach Funktionalität der Carbonsäure - einem oder mehreren Alkoholbausteinen aufgebaut, die jeweils - unabhängig voneinander - linear, verzweigt, gesättigt oder olefinisch ungesättigt sein können. Die Natur der Carbonsäurealkylester (a) unterliegt an sich keinen speziellen Beschränkungen.

Beispiele für geeignete Alkoholbausteine der Carbonsäurealkylester sind insbesondere Methanol, Ethanol, 1-Propanol und 2-Propanol.

Die Carbonsäurebausteine der Carbonsäurealkylester weisen vorzugsweise ein oder zwei Carboxylgruppen pro Molekül auf; dies bedeutet, daß als Carbonsäurebausteine der Carbonsäurealkylester vorzugsweise Monocarbonsäuren, also Carbonsäuren mit einer Carboxylgruppe pro Molekül, oder Dicarbonsäuren, also Carbonsäuren mit zwei Carboxylgruppen pro Molekül, eingesetzt werden.

Bei den Monocarbonsäure-Bausteinen sind die Fettsäuren bevorzugt, insbesondere solche mit 8 bis 24 C-Atomen. Bei den entsprechenden Verbindungen (a) handelt es sich mithin um Fettsäurealkylester.

Bei den Dicarbonsäure-Bausteinen sind die alpha-omega-Dicarbonsäuren bevorzugt. Bei den entsprechenden Verbindungen (a) handelt es sich mithin um Alkylester von alpha-omega-Dicarbonsäuren. Diese können als Vollester oder als Partialester vorliegen. Dabei sind Vollester bevorzugt.

Fettsäurealkylester von Fettsäuren mit 8 bis 24 C-Atomen sind als Verbindungen (a) bevorzugt. Beispiele für geeignete Fettsäurebausteine der Fettsäurealkylester sind etwa 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Ricinolsäure, 12-Hydroxystearinsäure, Arachinsäure, Gadoleinsäure, Behensäure und Erucasäure sowie deren technische Mischungen. Derartige Säuren fallen z.B. bei der Druckspaltung von natürlichen Fetten und Ölen oder bei der Reduktion von Aldehyden nach der Roelenschen Oxosynthese an. Bevorzugt als Fettsäurebausteine der Fettsäurealkylester sind technische (Spalt-)Fettsäuren mit 12 bis 18 Kohlenstoffatomen wie beispielsweise Kokos-, Palm-, Palmkern- oder Talgfettsäure sowie Isostearinsäure.

Als konkrete Beispiele für die vorgenannten Ester seien genannt: Caprinsäuremethylester, Laurinsäuremethylester, Myristinsäuremethylester, Palmitinsäuremethylester, Stearinsäuremethylester, Ölsäuresäuremethylester, Caprylsäureethylester, Caprinsäureethylester, Laurinsäureethylester, Myristinsäureethylester, Palmitinsäureethylester, Stearinsäureethylester und Ölsäuresäureethylester, genannt.

Als weitere konkrete Beispiele werden aus Kokosöl, Palmöl, Palmkernöl, Sojaöl, Rapsöl, Baumwollsaatöl, Olivenöl, Rindertalg, Fischöl und dergleichen abgeleitete Fettsäuremethylester und Fettsäureethylester genannt. Insbesondere bevorzugt werden aus Kokosöl, Palmöl und Palmkernöl abgeleitete Fettsäurealkylester. Diese Fettsäurealkylester können allein oder in Form von Kombinationen aus mindestens zwei dieser Ester verwendet werden.

In einer anderen Ausführungsform werden Alkylester von Dicarbonsäuren als Verbindungen (a) eingesetzt, vorzugsweise von alpha-omega-Dicarbonsäuren der Formel (I),

HOOC-(CH₂)n-COOH (I)

in der n für Zahlen von 1 bis 18, vorzugsweise 4 bis 10 steht. Typische Beispiele sind Malonsäure, Bernsteinsäure, Glutarsäure, Adipinsäure, Pimelinsäure, Korksäure, Azelainsäure, Sebacinsäure sowie 1,9-Nonandisäure, 1,16-Hexadecandisäure, 1,18-Octadecandisäure und 1,12-Dodecandisäure, wobei letztere bevorzugt ist.

In einer weiteren Ausführungsform werden Alkylester von Dimer- und/oder Trimerfettsäuren als Verbindungen (a) eingesetzt. Dimer- und/oder Trimerfettsäuren sind dem Fachmann bekannt. Dimerfettsäuren sind Carbonsäuren, die durch Oligomerisierung ungesättigter Carbonsäuren, in der Regel Fettsäuren wie Ölsäure, Linolsäure, Erucasäure und dergleichen, zugänglich sind. Üblicherweise erfolgt die Oligomerisierung bei erhöhter Temperatur in Gegenwart eines Katalysators aus etwa Tonerde. Die dabei erhaltenen Substanzen - Dimerfettsäuren technischer Qualität - stellen Gemische dar, wobei die Dimerisierungsprodukte überwiegen. Jedoch sind auch geringe Anteile höherer Oligomerer, insbesondere die Trimerfettsäuren, enthalten. Dimerfettsäuren sind handelsübliche Produkte und werden in verschiedenen Zusammensetzungen und Qualitäten angeboten.

Die Verbindungen (a) sind in den zur Hydrierung bestimmten Zusammensetzungen in einer Menge vorhanden, die im Bereich von 0,1 bis 99,3 Gew.-% - bezogen auf die Summe der Komponenten (a) bis (d) - liegt. Vorzugsweise werden die Verbindungen (a) in einer Menge eingesetzt, die im Bereich von 10 bis 70 Gew.-% und insbesondere 10 bis 60 Gew.-% liegt. Wie bereits ausgeführt gilt für binäre Systeme - also Systeme, die ausschließlich die Komponenten (a) und (d) enthalten -, die Bedingung, daß die Menge an Wasser (d) mindestens 2,1 Gew.-% beträgt und die Unter- und Obergrenzen der Menge an Carbonsäurealkylester (a) identisch sind und allein durch die Bedingung festgelegt sind, daß die Summe der Anteile der Komponenten (a) und (d) den Wert 100 Gew.-% ergibt

### Zu den Carbonsäuren (b)

In einer Ausführungsform handelt es sich bei den Verbindungen (b) um C₈₋₂₄-Fettsäuren.

In einer weiteren Ausführungsform handelt es sich bei den Verbindungen (b) um C₃₋₂₄-alpha-omega-Dicarbonsäuren.

In einer weiteren Ausführungsform handelt es sich bei den Verbindungen (b) um Dimer- und/oder Trimerfettsäuren. C₈₋₂₄-Fettsäuren sind als Verbindungen (b) besonders bevorzugt. Hierbei handelt es sich um Verbindungen der Formel (II),

R¹CO-OH (II)

in der R¹CO für einen linearen oder verzweigten, gesättigten oder ein- oder mehrfach olefinisch ungesättigten, gegebenenfalls hydroxyfunktionalisierten Acylrest mit 8 bis 24 Kohlenstoffatomen steht.

Die Natur der genannten Fettsäuren (b) unterliegt an sich keinen speziellen Beschränkungen. Beispiele für geeignete Fettsäuren (b) sind: 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Ricinolsäure, 12-Hydroxystearinsäure, Arachinsäure, Gadoleinsäure, Behensäure und Erucasäure sowie deren technische Mischungen. Derartige Säuren fallen z.B. bei der Druckspaltung von natürlichen Fetten und Ölen oder bei der Reduktion von Aldehyden nach der Roelen'schen Oxosynthese an. Bevorzugt als Fettsäuren (b) sind technische (Spalt-)Fettsäuren mit 12 bis 18 Kohlenstoffatomen wie beispielsweise Kokos-, Palm-, Palmkern- oder Talgfettsäure sowie Isostearinsäure.

Die Fettsäuren (b) sind in den zur Hydrierung bestimmten Zusammensetzungen vorzugsweise in einer Menge vorhanden, die im Bereich von 0,1 bis 95 Gew.-% - bezogen auf die Summe der Komponenten (a) bis (d) - liegt. Bevorzugt ist insbesondere ein Bereich von 20 bis 80 Gew.-% und ganz besonders von 20 bis 60 Gew.-%.

### Zu den Alkanolen (c)

Bei den Verbindungen (c) handelt es sich um Alkohole mit 1 bis 3 C-Atomen und einer OH-Gruppe pro Molekül. Dies sind Methanol, Ethanol, 1-Propanol und 2-Propanol, Methanol ist als Alkanol (c) besonders bevorzugt.

Die Alkanole (c) sind in den zur Hydrierung bestimmten Zusammensetzungen vorzugsweise in einer Menge vorhanden, der im Bereich von 0,1 bis 90 Gew.-% - bezogen auf die Summe der Komponenten (a) bis (d) - liegt. Bevorzugt ist ein Bereich von 1 bis 65 Gew.-% und insbesondere 1 bis 50 Gew.-%.

### Die Komponente (d): Wasser

Wasser (d) ist in den zur Hydrierung bestimmten Zusammensetzungen einer Menge vorhanden, der im Bereich von 1,5 bis 20 Gew.-% ϕ - bezogen auf die Summe der Komponenten (a) bis (d) - liegt, wobei die oben genannten weiteren Maßgaben zu beachten sind. Bevorzugt ist für die Menge an Komponente (d) - bezogen auf die Summe der Komponenten (a) bis (d) - ein Bereich von 2,0 bis 15 Gew.-% und ganz besonders 3,0 bis 15 Gew.-%.

Vorzugsweise beträgt die Menge an Wasser (d) - bezogen auf die Menge an Komponente (a) - mindestens 3,0 Gew.-% und insbesondere mindestens 3,4 Gew.-%. Ganz besonders bevorzugt ist es, daß die Menge an Wasser (d) - bezogen auf die Menge an Komponente (a) - mindestens 4,0 Gew.-% beträgt.

### Zur Hydrierung

Die Hydrierung kann in an sich bekannter Weise, also bei Temperaturen im Bereich von 150 bis 300 °C und Drucken im Bereich von 20 bis 300 bar erfolgen. Temperaturen im Bereich von 220 bis 240 °C und Drucke im Bereich von 200 bis 280 bar sind dabei bevorzugt. Die Wasserstoffmenge, die während der Kontakthydrierung zugeführt wird, unterliegt an sich keinen besonderen Beschränkungen. Sie orientiert sich im wesentlichen an den dem Fachmann bekannten Mengen, die bei der Hydrierung von Fettsäuren bzw. Fettsäuremethylostern eingesetzt werden.

Generell ist im Sinne der erfindungsgemäßen Lehre sicherzustellen, daß eine Mischung der Komponenten (a) bis (d) - wie bereits oben hinsichtlich Art und Menge der einzelnen Komponenten näher beschrieben - als binäres, ternäres oder - was besonders bevorzugt ist - quaternäres System der Hydrierung zugeführt wird. Die zur Hydrierung einzusetzenden Mischungen der Komponenten (a) bis (d) entstehen also nicht erst während der Hydrierung, sondern liegen bereits vor Beginn der Hydrierung vor und werden der Hydrierung zugeführt. Wenn also beispielsweise eine Suspensionshydrierung durchgeführt wird, so gilt, daß dort, wo das zu hydrierende Gut in Kontakt mit Wasserstoff und dem Hydrierkatalysator kommt, die Mischung der Komponenten (a) bis (d) in der oben näher beschriebenen Zusammensetzung vorliegt.

Die Hydrierung kann an sich batchweise oder kontinuierlich durchgeführt werden. Erfindungsgemäß ist jedoch die kontinuierliche Hydrierung bevorzugt. Dabei unterliegen die zum Einsatz kommenden Hydrierreaktoren unterliegen an sich keinerlei Beschränkungen, solange sie sich zur Ausführung der Kontakthydrierung eignen. Es können somit üblicherweise eingesetzte allgemein bekannte Reaktoren verwendet werden. Als Beispiele für derartige Reaktoren werden Fließbettreaktoren, in denen die Kontakthydrierung unter Verwendung von in Flüssigkeiten geführten Katalysatoren vorgenommen wird, Bewegtbettreaktoren, in denen die Kontakthydrierung derart vorgenommen wird, dass die flüssigen Ausgangsstoffe zugeführt werden, während die gesamte Katalysatorschicht schwerkraftbedingt allmählich nach unten fällt, und Festbettreaktoren genannt, in denen die Kontakthydrierung derart vorgenommen wird, dass die flüssigen Ausgangsstoffe zugeführt werden, wobei die Katalysatoren in den Reaktor eingefüllt und dort immobilisiert sind.

Festbettreaktoren sind im Rahmen der vorliegenden Erfindung zur Hydrierung bei weitem bevorzugt. Vorzugsweise arbeitet man dabei in Rohr- oder Schachtreaktoren, in denen die Katalysatoren in stückiger Form, also beispielsweise als Pellets angeordnet sind. Auch beim Einsatz von Rohr- oder Schachtreaktoren gilt, daß dort, wo das zu hydrierende Gut in Kontakt mit Wasserstoff und dem Hydrierkatalysator kommt, die Mischung der Komponenten (a) bis (d) in der oben näher beschriebenen Zusammensetzung vorliegt.

Als Katalysatoren eignen sich handelsübliche oxidische Kupfer-Zink-, Aluminium-Zink-, Kupfer-Aluminium- oder Kupfer-Chrom-Kontakte, insbesondere Cu-Chromit-Katalysatoren, die beispielsweise von den Firmen Engelhard oder Süd-Chemie bezogen werden können, wobei letztere wegen ihrer besonderen Säurebeständigkeit bevorzugt sind. Diese Kontakte können mit weiteren Metallen, wie beispielsweise Barium, Mangan, Magnesium, Cadmium oder Cer dotiert sein. Aus Gründen des Korrosionsschutzes empfiehlt es sich ferner, die Hydrieranlage in Edelstahl auszuführen.

Im Rahmen des erfindungsgemäßen Verfahrens ist es besonders bevorzugt, die Hydrierung in Gegenwart eines stückigen Cu-Chromit-Katalysators durchzuführen und zwar bei kontinuierlicher Fahrweise in einem Rohr- oder Schachtreaktor in Rieselfahrweise. Dabei kann es gewünscht sein, zwei oder mehrere dieser Reaktoren hintereinanderzuschalten.

In einer Ausführungsform wird die Hydrierung bei einer Leerrohrgeschwindigkeit des Gasstromes - bezogen auf die Gasdichte am Eintritt - im Bereich von 2,5 bis 15 cm/s und insbesondere 4 bis 12 cm/s durchgeführt. Unter Leerrohrgeschwindigkeit (superficial gas velocity) wird dabei - wie in Fachkreisen allgemein üblich - das Verhältnis aus Volumenstrom und Querschnittsfläche des Reaktors verstanden. Dabei wird der Volumenstrom für die Bedingungen am Reaktoreintritt eingesetzt. Für die Querschnittsfläche wird dabei nicht der freie Strömungsquerschnitt der Katalysatorschüttung, sondern der gesamte innere Querschnitt des Rohr- bzw. des Schachtreaktors angesetzt.

### Beispiele

### Eingesetzte Substanzen

**Fettsäuremethylester (FSMe):** Mischung von Laurinsäuremethylester und Myristinsäuremethylester im Gewichtsverhältnis 70 : 30 ("Edenor ME C12 70 " der Fa. Cognis Deutschland GmbH&Co. KG); Molgewicht = 222,8 g/mol; Dichte = 868,8 kg/m³ **Fettsäure (FS):** Mischung von Laurinsäure und Myristinsäure im Gewichtsverhältnis 70 : 30 ("Edenor C12 70" der Fa. Cognis Deutschland GmbH&Co. KG); Molgewicht = 208,7 g/mol; Dichte = 846,8 kg/m³
**Wasser:** Molgewicht = 18,0 g/mol
**Methanol (MeOH):** Molgewicht = 32,0 g/mol

### Allgemeines

Im Folgenden bedeuten:
- FSMe = Fettsäuremethylester der oben genannten Zusammensetzung
- FS = Fettsäure der oben genannten Zusammensetzung
- MeOH = Methanol
- H₂O = Wasser
- V1, V2 usw = Versuch 1, Versuch 2, usw.
- Die Versuche V1 bis V4 sowie V13 dienen dem Vergleich. Die übrigen Versuche sind erfindungsgemäß.

### Durchführung der Versuche

### Allgemeine orientierende Beschreibung

In einem Vorlagebehälter wurden die jeweiligen Einsatzstoffe vorgelegt, in flüssige Form überführt und durch Rühren homogenisiert. Vom Vorlagebehälter aus gelangte die flüssige Vormischung in einen rohrförmigen Wärmetauscher, in dem - je nach eingesetzten Substanzen - eine in-situ-Veresterung und/oder Hydrolyse stattfinden konnte. Diese Reaktion erfolgte ohne die Gegenwart eines Katalysators. Der Transport der Flüssigkeit geschah mittels einer Membranpumpe. Unmittelbar vor dem Eintritt in den Hydrierreaktor wurde die zu hydrierende Flüssigkeit mit Wasserstoffgas zusammengeführt. Vor dieser Stelle, an der Flüssigkeit und Gas zusammengeführt wurden, befand sich eine **Probenahmestelle**, an der die Zusammensetzung der zu hydrierenden flüssigen Phase festgestellt wurde (zu hydrierendes Gut; siehe Tabelle 1). Als Hydrierrektor diente ein mit einem handelsüblichen oxidischem Kupfer-Chrom-Katalysator gefüllter Schachtreaktor, der von dem zu hydrierenden Gut (Flüssigkeit) und Wasserstoff (Gas) von oben nach unten durchströmt wurde. Gas und Flüssigkeit wurden am Austritt des Reaktors in zwei seriell geschalteten Abscheidern getrennt, die jeweils in einzelne Niederdruckbehälter entleert werden konnten. Die bei der Hydrierung angefallenen Zusammensetzungen wurden nasschemisch und gaschromatographisch analysiert (Produktanalyse; siehe Tabelle 2).

### Einzelheiten der Beschreibung

In einem elektrisch beheizbaren **Vorlagebehälter** wurden die jeweiligen Einsatzstoffe (siehe oben unter "eingesetzte Substanzen") in unterschiedlichen Mischungsverhältnissen vorgelegt. Die Mischungen wurden so temperiert, dass sie flüssig vorlagen. Hierzu wurden Temperaturen im Bereich von 40 bis 75°C eingestellt Der Vorlagebehälter verfügte einen elektrisch betriebenen Rührer, mit dem die flüssigen Einsatzstoffe jeweils homogenisiert wurden. Die im Vorlagebehälter befindlichen flüssigen und homogenisierten Einsatzstoffe wurden mittels einer Membranpumpe über eine Flüssigkeitsleitung angesaugt und auf 250 bar gebracht und mittels eines mit Thermalöl (*Malotherm SH)* temperierten rohrförmigen Wärmetauschers auf eine Temperatur im Bereich von 240 bis 245 °C gebracht. Das Thermalöl, das den Wärmetauscher außen durchströmte, wurde mit einem Heizgerät von *Single* temperiert, das eine Heizleistung 18 KW und eine Kühlleistung von 69,6 kW aufwies (Typ *STO 1-18-80-D2, PK SM11*). Der Mindestvolumenstrom des Thermalöls betrug 1 m³/h.

Bevor die zu hydrierende Flüssigkeit mit der Gasphase (Wasserstoff) zusammengeführt wurde, wurde vor Eintritt in den Hydrierreaktor eine flüssige Probe entnommen und analysiert (**Probenahmestelle** mit der Schaltung: Kondensator, Abscheider, Spülvorrichtung). Die Zusammensetzung der zu hydrierenden Flüssigkeit für jeden Versuch kann **Tabelle 1** entnommen werden.

Unmittelbar nach der Probenahmestelle wurden Gas (Wasserstoff) und Flüssigkeit zusammengeführt und als zweiphasiger Strom in den Reaktor geleitet.

Als Hydrierrektor diente ein Schachtreaktor mit einem Durchmesser von 30 mm und einer Länge von 2,4 m (V_{Reaktor} = 1,7 Liter). Dieser Reaktor war mit einem kommerziell erhältlichen oxidischem Kupfer-Chrom-Katalysator der Firma Engelhard (Katalysator-Typ: Cu-1986T 1/8) beschickt worden, wobei die Aktivierung des Katalysators reduktiv erfolgte (einmaliger Vorgang). Gas und Flüssigkeit durchströmten den Hydrierreaktor durch die Katalysatorschüttung in der gleichen Richtung, nämlich von oben nach unten, wobei sich die Flüssigkeit als dünner Film über die äußere Oberfläche der Schüttung ausbreiteten konnte (sogenannte Rieselbett-Fahrweise).

Der Reaktor wurde über einen zweiten Thermalölkreislauf temperiert, der unabhängig von demjenigen des Wärmetauschers am Reaktoreintritt geregelt wurde. Der Reaktor wurde unabhängig vom Wärmetauscher mit Thermalöl beheizt, das ebenfalls im Kreis geführt wurde und mit Hilfe eines zweiten Heizgerätes des oben genannten Typs temperiert wurde. Innerhalb einer inerten Deckschicht aus feinen Glaskugeln (d = 2,5 mm), die zugleich eine gute Verteilung der Flüssigkeit über dem Querschnitt hervorriefen, konnte das kalt eingespeiste Gas (Wasserstoff) auf Reaktionstemperatur gebracht werden.

Die Hydrierung im Reaktor erfolgte bei einer Temperatur von 240 °C und einem Wasserstoffdruck von 250 bar, sowie einem Durchsatz von 850 - 1000 g/h (bei den Versuchen V1 bis V22 sowie den Versuchen V43 bis V53 betrug der Durchsatz 1000 g/h, bei den Versuchen V23 bis V26 betrug der Durchsatz 950 g/h, bei den Versuchen V27 bis V29 betrug der Durchsatz 900 g/h und bei den Versuchen V30 bis V42 betrug der Durchsatz 850 g/h).

Für die bei der Hydrierung erhaltenen Rohalkohole wurden einerseits die nasschemischen Kennzahlen bestimmt. Darüber hinaus wurde eine gaschromatographische Analyse der Nebenprodukte durchgeführt. Die Ergebnisse der ermittelten analytischen Daten sind in **Tabelle 2** zusammengefasst.

Den Tabellen kann jeweils in der ersten Spalte die Versuchsnummer (Versuche V1 bis V53) entnommen werden. Die Versuche wurden - entsprechend der laufenden Numerierung der Versuche - chronologisch hintereinander durchgeführt. Je Versuch wurde die geschilderte Anlage jeweils 7 Stunden betrieben und dann die Daten gemäß den Tabellen bestimmt. Darüber hinaus wurde zu Kontrollzwecken in regelmäßigen Zeitabständen ein Versuch zwischengeschaltet, bei dem als Rohstoff (= als zu hydrierendes Gut) ausschließlich Fettsäuremethylester FSMe (wie in Versuch V1) eingesetzt wurde. Dabei zeigte sich, daß der Daten der Produktanalyse den Daten von Versuch V1 entsprachen.

### Dosierung der Mengenströme

Die Flüssigkeit wurde mit einer Membranpumpe gefördert, deren Durchsatz eine Variation im Bereich von 0,5 und 5 kg gestattete (Typ *LEWA EK1*). Der flüssige Durchsatz wurde mit einem Messgerät von *Micro Motion* erfasst, dessen Messung auf der Analyse der durchflussabhängigen Coriolis-Kraft beruht (Schwingungsanalyse mit einem Sensor vom Typ *ELITE CMF010N*, Messgenauigkeit +/- 0,25%). Über einen PI-Regler wurde der Durchsatz der Pumpe auf den gewünschten Wert eingestellt.

Der Gasstrom wurde über ein Dosier-Ventil gesteuert, das sich in der Austrittsleitung befand. Über diesem Dosierventil lag eine Druckdifferenz von ca. 250 bar an. Auf der Niederdruckseite unmittelbar hinter dem Dosierventil wurde der Gasstrom mit Hilfe eines Rotameters erfasst (Schwebekörper-Durchflussmessung).

**Tabelle 1: Zur Hydrierung eingesetzte Mischungen**

| Versuch | in Gew.-% | | | | in mol-% | | | |
|---|---|---|---|---|---|---|---|---|
| | FSMe | FS | H2O | MeOH | FSMe | FS | H2O | MeOH |
| V8 | 52,6 | 25,8 | 4,2 | 17,5 | 20,7 | 10,9 | 20,7 | 47,8 |
| V9 | 44,4 | 32,4 | 4,6 | 18,6 | 16,7 | 13,0 | 21,4 | 48,8 |
| V10 | 37,7 | 37,7 | 5,0 | 19,6 | 13,6 | 14,6 | 22,6 | 49,2 |
| V11 | 29,2 | 42,6 | 7,4 | 20,8 | 9,4 | 14,7 | 29,3 | 46,6 |
| V12 | 37,0 | 30,3 | 13,0 | 19,7 | 10,1 | 8,8 | 43,8 | 37,3 |
| V13 | 94,6 | 4,7 | 0,0 | 0,7 | 90,4 | 4.8 | 0,0 | 4,8 |
| V14 | 83,3 | 8,7 | 6,7 | 1,3 | 45,0 | 5,0 | 45,0 | 5,0 |
| V15 | 81,7 | 8,5 | 8,4 | 1,3 | 40,0 | 4,4 | 51,1 | 4,4 |
| V16 | 79,4 | 8,3 | 11,1 | 1,3 | 34,0 | 3,8 | 58,5 | 3,8 |
| V17 | 79,2 | 12,4 | 6,4 | 1,8 | 42,8 | 7,2 | 42,8 | 7,2 |
| V18 | 73,2 | 16,5 | 7,8 | 2,5 | 35,8 | 8,6 | 46,9 | 8,6 |
| V19 | 70,6 | 16,5 | 10,3 | 2,5 | 30,2 | 7,5 | 54,7 | 7,5 |
| V20 | 72,1 | 19,2 | 5,8 | 2,9 | 38,9 | 11,1 | 38,9 | 11,1 |
| V21 | 72,6 | 17,0 | 7,7 | 2,6 | 36,6 | 8,9 | 46,7 | 8,9 |
| V22 | 64,6 | 22,1 | 9,9 | 3,4 | 27,6 | 10,1 | 52,2 | 10,1 |
| V23 | 66.9 | 26,7 | 2,3 | 4,1 | 43,8 | 18,7 | 18,8 | 18,7 |
| V24 | 64,4 | 26,3 | 5,2 | 4,0 | 34,8 | 15,2 | 34,8 | 15,2 |
| V25 | 63,4 | 25,7 | 7,0 | 3,9 | 31,0 | 13,4 | 42,2 | 13,4 |
| V26 | 58.7 | 27,6 | 9,4 | 4,2 | 25,1 | 12,6 | 49,6 | 12,6 |
| V27 | 58,2 | 34,5 | 2,0 | 5,3 | 37,1 | 23,5 | 15,9 | 23,5 |
| V28 | 58,3 | 32,1 | 4,7 | 4,9 | 31,5 | 18,5 | 31,5 | 18,5 |
| V29 | 51,8 | 43,3 | 6,0 | 5,6 | 25,3 | 19,1 | 36,4 | 19,1 |
| V30 | 43,3 | 42,1 | 8,1 | 6,5 | 18,5 | 19,2 | 43,0 | 19,2 |
| V31 | 72,5 | 10,9 | 2,8 | 13,8 | 33,8 | 6,4 | 16,1 | 44,7 |
| V32 | 68,3 | 12,5 | 5,5 | 13,7 | 27,9 | 5,5 | 27,9 | 38,8 |
| V33 | 64,3 | 15,1 | 6,8 | 13,9 | 24,6 | 6,2 | 32,3 | 36,9 |
| V34 | 62,6 | 14,7 | 9,2 | 13,5 | 21,9 | 5,5 | 39,7 | 32,9 |
| V36 | 69,3 | 13,7 | 2,9 | 14,2 | 31,8 | 6,7 | 16,4 | 45,2 |
| V36 | 64,4 | 16,2 | 6,2 | 14,2 | 26,3 | 7,1 | 26,3 | 40,4 |
| V37 | 60,9 | 19,0 | 6,5 | 14,5 | 23,0 | 7,8 | 30,7 | 38,6 |
| V38 | 59,4 | 17,7 | 8,9 | 14,0 | 20,8 | 6,6 | 38,6 | 34,0 |
| V39 | 67,5 | 7,1 | 2,7 | 22,7 | 25,3 | 2,8 | 12,7 | 59,2 |
| V40 | 63,8 | 8,7 | 5,2 | 22,4 | 21,8 | 3,2 | 21,8 | 53,2 |
| V41 | 62,9 | 8,6 | 6,5 | 22,0 | 20,5 | 3,0 | 26,4 | 50,0 |
| V42 | 60,4 | 9,3 | 8,6 | 21,7 | 18,6 | 3.0 | 32,4 | 46,1 |
| V43 | 63,9 | 10,2 | 2,7 | 23,1 | 23,7 | 4,1 | 12,6 | 59,6 |
| V44 | 60,0 | 12,3 | 4,9 | 22,9 | 20.5 | 4,5 | 20,5 | 54,5 |
| V45 | 69,9 | 11,3 | 6,3 | 22,5 | 19,6 | 4,0 | 25,5 | 51,0 |
| V46 | 54,2 | 15,2 | 8,1 | 22,6 | 16,6 | 5,0 | 30,5 | 48,0 |
| V47 | 61,8 | 12,8 | 2,9 | 22,6 | 23,1 | 5,1 | 13,2 | 58,6 |
| V48 | 58,4 | 14,5 | 4,7 | 22,4 | 20,3 | 5,4 | 20,3 | 54,1 |
| V49 | 55,9 | 15,8 | 6,0 | 22,3 | 18,5 | 5,6 | 24,5 | 51,4 |
| V50 | 46,1 | 24,3 | 7,4 | 23,2 | 14,0 | 8,0 | 28,2 | 49,5 |
| V51 | 53,3 | 25,1 | 4,3 | 17,3 | 21,0 | 10,5 | 21,0 | 47,5 |
| V52 | 41,3 | 41,3 | 3,3 | 14,1 | 18,4 | 19,6 | 18,4 | 43,6 |
| V53 | 16,1 | 69,9 | 1,3 | 12,7 | 8,2 | 38,3 | 8,2 | 45,2 |

**Tabelle 2: Produktanalyse**

| Versuch | SZ | VZ | H₂O | COZ | POZ | KW | s.-Alk. | 1.-Nlk. | Ether |
|---|---|---|---|---|---|---|---|---|---|
| V8 | 0,04 | 1,12 | 0,71 | 32 | < 0,05 | 0,59 | 0,07 | 0,01 | 0.44 |
| V9 | 0,04 | | 0,58) | 36 | < 0,05 | 0,62 | 0,06 | 0,01 | 0,48 |
| V10 | 0,04 | 1,25 | 0,46 | 37 | <0.06 | 0.52 | 0,06 | 0,01 | 0,40 |
| V11 | 0,04 | 1,23 | 1,20 | 36 | < 0,05 | 0.43 | 0,06 | 0 | 0,41 |
| V12 | 0,04 | 1,24 | 1,06 | 39 | < 0,05 | 0,38 | 0,06 | 0 | 0,45 |
| V13 | 0,02 | 1,06 | 0,06 | 43 | 0,06 | 1,64 | 0,17 | 0.05 | 0,38 |
| V14 | 0,03 | 1,17 | 0,39 | 41 | 0,06 | 0,54 | 0,07 | 0,01 | 0,35 |
| V16 | 0,04 | 1,32 | 0,50 | 41 | 0,05 | 0,55 | 0,07 | 0,01 | 0,37 |
| V16 | 0,02 | 1,27 | 0,36 | 46 | 0,05 | 0,54 | 0,07 | 0,01 | 0,37 |
| V17 | 0,03 | 1,19 | 0,32 | 44 | 0.05 | 0,54 | 0,07 | 0,01 | 0,37 |
| V18 | 0,03 | 1,19 | 0,60 | 44 | 0,05 | 0,53 | 0,07 | 0,01 | 0,37 |
| V19 | 0,03 | 1,34 | 2,24 | 46 | 0,05 | 0,52 | 0,07 | 0 | 0,37 |
| V20 | 0,02 | 1,36 | 0,34 | 47 | 0,05 | 0,55 | 0,06 | 0,01 | 0,33 |
| V21 | 0,03 | 1,41 | 0,69 | 49 | 0,06 | 0,47 | 0,06 | 0 | 0,34 |
| V22 | 0,03 | 1,32 | 0,75 | 51 | 0,05 | 0,44 | 0,07 | 0 | 0,33 |
| V23 | 0,02 | 1,25 | 0,23 | 50 | 0,06 | 0,75 | 0,07 | 0,01 | 0,31 |
| V24 | 0,02 | 1,20 | 0,29 | 48 | 0,05 | 0,85 | 0,07 | 0,01 | 0,33 |
| V25 | 0,02 | 1,42 | 0,14 | 44 | 0,05 | 0,62 | 0,07 | 0,01 | 0,36 |
| V26 | 0,04 | 1,90 | 0,67 | 60 | 0,05 | 0,37 | 0,05 | 0 | 0,32 |
| V27 | 0,02 | 1,25 | 0,17 | 58 | 0,05 | 0,73 | 0,07 | 0,01 | 0,31 |
| V28 | 0,04 | 1,50 | 0,38 | 39 | 0,05 | 0,55 | 0,06 | 0,01 | 0,34 |
| V29 | 0,02 | 1,66 | 0,78 | 42 | 0,05 | 0,49 | 0,06 | 0 | 0,33 |
| V30 | 0,02 | 1,83 | 0,59 | 51 | 0,05 | 1,05 | 0,04 | 0 | 0.40 |
| V31 | 0,02 | 0,98 | 0,11 | 42 | 0,05 | 1,05 | 0,12 | 0,03 | 0,34 |
| V32 | 0,02 | 1,00 | 0,36 | 42 | 0,05 | 0,76 | 0.10 | 0,01 | 0,36 |
| V33 | 0,02 | 1,02 | 0,42 | 42 | 0,05 | 0,71 | 0,10 | 0,01 | 0,35 |
| V34 | 0,02 | 0,94 | 0,40 | 48 | 0,06 | 0,58 | 0,09 | 0,01 | 0,36 |
| V36 | 0,02 | 1,05 | 0,36 | 48 | 0,05 | 0,97 | 0.11 | 0,01 | 0,32 |
| V36 | 0,02 | 0,91 | 0,41 | 49 | 0,05 | 0,73 | 0,10 | 0,01 | 0,34 |
| V37 | 0,04 | 0,83 | 0,77 | 51 | 0,06 | 0,64 | 0.10 | 0.01 | 0,36 |
| V38 | 0,02 | 0,87 | 0,74 | 55 | 0,05 | 0,58 | 0,09 | 0,01 | 0,36 |
| V39 | 0,02 | 0,80 | 0.21 | 47 | 0,05 | 0,97 | 0,13 | 0,02 | 0,36 |
| V40 | 0,02 | 0,89 | 0,58 | 44 | 0,05 | 0,72 | 0,08 | 0.01 | 0,36 |
| V41 | 0,02 | 0,84 | 0,58 | 44 | 0,05 | 0,72 | 0,12 | 0,01 | 0,36 |
| V42 | 0,02 | 0,65 | 0,41 | 48 | 0,05 | 0,65 | 0,12 | 0.01 | 0,39 |
| V43 | 0,02 | 0,94 | 0,21 | 48 | 0,05 | 0.8 | 0,10 | 0,02 | 0,30 |
| V44 | 0,02 | 0,79 | 0,30 | 45 | 0,05 | 0,65 | 0,09 | 0,01 | 0,31 |
| V45 | 0,02 | 0,94 | 0,25 | 44 | 0,05 | 0,64 | 0,10 | 0,01 | 0,33 |
| V46 | 0,02 | 0,85 | 0,40 | 50 | 0,06 | 0,5 | 0,08 | 0,01 | 0,33 |
| V47 | 0,02 | 1,03 | 0,22 | 50 | 0,05 | 0,73 | 0,10 | 0,01 | 0,29 |
| V48 | 0,02 | 1,09 | 0,61 | 48 | 0,06 | 0,61 | 0,10 | 0,01 | 0,29 |
| V49 | 0,02 | 1,08 | 0,44 | 55 | 0,06 | 0,5 | 0,09 | 0,01 | 0,30 |
| V50 | 0,02 | 1,03 | 0,29 | 52 | 0,05 | 0,46 | 0,09 | 0,01 | 0,33 |
| V51 | 0,02 | 1,19 | 0,33 | 58 | 0,05 | 0,49 | 0,07 | 0,01 | 0,27 |
| V52 | 0,02 | 1,18 | 0,38 | 57 | 0,05 | 0,46 | 0.06 | 0,01 | 0,25 |
| V53 | 0,02 | 3,55 | 0,24 | 50 | 0,05 | 0,44 | 0,04 | 0,01 | 0,26 |

In Tabelle 2 bedeuten:
**SZ** = Säurezahl (acid value), bestimmt nach DIN-ISO 660
**VZ** = Verseifungszahl (saponification value), bestimmt nach DIN-ISO 3657
**H₂O** = Wassergehalt (in Gew.-%), bestimmt nach DIN-ISO 760
**COZ** = Carbonylzahl (in ppm), bestimmt nach DGF CV 18
**POZ** = Peroxidzahl (in mval), bestimmt nach DGF C-VI 6a
**KW** = Kohlenwasserstoffe (Definition siehe oben), gemessen mittels Gaschromatographie (Flächenprozent)
**s.Alk.** = sekundäre Alkohole (Definition siehe oben), gemessen mittels Gaschromatographie (Flächenprozent)
**i.Alk.** = Iso-Alkohole (Definition siehe oben), gemessen mittels Gaschromatographie (Flächenprozent)
**Ether** = Ether (Dialkylether mit 24 bis 30 C-Atomen), gemessen mittels Gaschromatographie (Flächenprozent)

## Patentansprüche

1. Verfahren zur Herstellung von Fettalkoholen, indem man eine Mischung enthaltend
(a) 10 bis 70 Gew.-% ein oder mehrere C₈₋₂₄-Fettsäure-C₁₋₃-alkylester,
(b) 20 bis 80 Gew.-% ein oder mehrere C₈₋₂₄-Fettsäuren,
(c) 1 bis 65 Gew.-% ein oder mehrere C₁₋₃-Alkanole und
(d) 1,5 bis 20 Gew.-% Wasser,
wobei sich die Gew.-% Angaben der Komponenten (a) bis (d) jeweils auf die Summe der Komponenten (a) bis (d) beziehen,
in Gegenwart eines heterogenen stückigen Katalysators kontinuierlich hydriert,
mit der zusätzlichen Maßgabe, dass
• die Summe der Anteile der Komponenten (a) bis (d) den Wert 100 Gew.-% ergibt und
• die Menge an Wasser (d) - bezogen auf die Menge an C₈₋₂₄-Fettsäure-C₁₋₃-alkylester (a) - mindestens 3,4 Gew.-% beträgt.

2. Verfahren nach Anspruch 1, wobei die zu hydrierende Mischung enthält:
(a) 10 bis 60 Gew.-% ein oder mehrere C₈₋₂₄-Fettsäuremethylester,
(b) 20 bis 60 Gew.-% ein oder mehrere C₈₋₂₄-Fettsäuren,
(c) 1 bis 60 Gew.-% Methanol und
(d) 2,0 bis 15 Gew.-% Wasser.

3. Verfahren nach Anspruch 1 oder 2, wobei man die zu hydrierende Mischung der Komponenten (a) bis (d) herstellt, indem man ein oder mehrere C₈₋₂₄-Fettsäuren (b) mit Methanol (c) mischt und bei erhöhtem Druck und erhöhter Temperatur, in Abwesenheit eines Katalysators miteinander im Sinne einer zumindest partiellen Veresterung umsetzt.

4. Verfahren nach Anspruch 3, wobei man bei der Herstellung der Mischung der Komponenten (a) bis (d) bei einem Druck von mindestens 60 bar und einer Temperatur von mindestens 200 °C arbeitet.

5. Verfahren nach Anspruch 3, wobei man bei der Herstellung der Mischung der Komponenten (a) bis (d) bei einem Druck von mindestens 80 bar und einer Temperatur von mindestens 240 °C arbeitet.

6. Verfahren nach Anspruch 1 oder 2, wobei man die zu hydrierende Mischung der Komponenten (a) bis (d) herstellt, indem man ein oder mehrere C₈₋₂₄-Fettsäuren (b) mit Methanol (c) mischt und diese Mischung - gegebenenfalls bei erhöhtem Druck und erhöhter Temperatur - in einem Veresterungsreaktor, der einen festen, immobilisierten Veresterungskatalysator enthält, einer zumindest partiellen Veresterung unterwirft.

7. Verfahren nach einem der Ansprüche 1 bis 6, wobei man die Hydrierung in Gegenwart eines heterogenen stückigen Cu-Chromit-Katalysators durchführt.

8. Verfahren nach Anspruch 1 bis 7, wobei man die Hydrierung in einem Festbettreaktor durchführt.

9. Verfahren nach Anspruch 7 oder 8, wobei man die Hydrierung in einem Rohrbündel- oder einem Schachtreaktor in Rieselfahrweise durchführt.

## Claims

1. A process for the production of fatty alcohols by continuously hydrogenating a mixture containing
(a) 10 to 70% by weight of one or more C₈₋₂₄ fatty acid-C₁₋₃-alkyl esters,
(b) 20 to 80% by weight of one or more C₈₋₂₄ fatty acids,
(c) 1 to 65% by weight of one or more C₁₋₃ alkanols and
(d) 1.5 to 20% by weight of water,
the percentages by weight of components (a) to (d) each being based on the sum of components (a) to (d),
in the presence of a heterogeneous particulate
catalyst,
with the additional proviso that
• the sum of the proportions of components (a) to (d) amounts to 100% by weight and
• the quantity of water (d) - based on the quantity of C₈₋₂₄ fatty acid-C₁₋₃ alkyl ester (a) - amounts to at least 3.4% by weight.

2. A process as claimed in claim 1, wherein the mixture to be hydrogenated comprises:
(a) 10 to 60% by weight of one or more C₈₋₂₄ fatty acid methyl esters,
(b) 20 to 60% by weight of one or more C₈₋₂₄ fatty acids,
(c) 1 to 60% by weight of methanol and
(d) 2.0 to 15% by weight of water.

3. A process as claimed in claim 1 or 2, wherein the mixture of components (a) to (d) to be hydrogenated is prepared by mixing one or more C₈₋₂₄ fatty acids (b) with methanol (c) and reacting them with one another by at least partial esterification at elevated temperature and pressure in the absence of a catalyst.

4. A process as claimed in claim 3, wherein the mixture of components (a) to (d) is prepared under a pressure of at least 60 bar and at a temperature of at least 200°C.

5. A process as claimed in claim 3, wherein the mixture of components (a) to (d) is prepared under a pressure of at least 80 bar and at a temperature of at least 240°C.

6. A process as claimed in claim 1 or 2, wherein the mixture of components (a) to (d) to be hydrogenated is prepared by mixing one or more C₈₋₂₄ fatty acids (b) with methanol (c) and subjecting the resulting mixture to at least partial esterification, optionally at elevated temperature and pressure, in an esterification reactor containing a solid, fixed esterification catalyst.

7. A process as claimed in any of claims 1 to 6, wherein the hydrogenation is carried out in the presence of a heterogeneous particulate copper chromite catalyst.

8. A process as claimed in claims 1 to 7, wherein the hydrogenation is carried out in a fixed-bed reactor.

9. A process as claimed in claim 7 or 8, wherein the hydrogenation is carried out in the trickle phase in a tube-bundle or shaft reactor.

## Revendications

1. Procédé pour la préparation d'alcools gras en soumettant un mélange contenant
(a) de 10 à 70 % en poids d'un ou plusieurs esters alkyliques en C₁₋₃ d'acides gras en C₈₋₂₄,
(b) de 20 à 80 % en poids d'un ou plusieurs acides gras en C₈₋₂₄,
(c) de 1 à 65 % en poids d'un ou plusieurs alcanols en C₁₋₃ et
(d) de 1,5 à 20 % en poids d'eau,
les pourcentages en poids des composants (a) à (d) se rapportant chacun à la somme des composants (a) à (d),
à une hydrogénation en continu en présence d'un catalyseur hétérogène en morceaux,
étant en outre précisé que
• la somme des proportions des composants (a) à (d) donne la valeur de 100 % en poids et
• la quantité d'eau (d) - par rapport à la quantité d'ester(s) alkylique(s) en C₁₋₃ d'acides gras en C₈-₂₄ (a) - représente au moins 3,4 % en poids.

2. Procédé selon la revendication 1, dans lequel le mélange à hydrogéner contient:
(a) de 10 à 60 % en poids d'un ou plusieurs esters méthyliques d'acides gras en C₈₋₂₄,
(b) de 20 à 60 % en poids d'un ou plusieurs acides gras en C₈₋₂₄,
(c) de 1 à 60 % en poids de méthanol et
(d) de 2,0 à 15 % en poids d'eau.

3. Procédé selon la revendication 1 ou 2, dans lequel on prépare le mélange à hydrogéner des composants (a) à (d) en mélangeant un ou plusieurs acides gras en C₈₋₂₄ (b) avec du méthanol (c) et en les faisant réagir entre eux sous une pression élevée et à haute température, en absence d'un catalyseur, selon une estérification au moins partielle.

4. Procédé selon la revendication 3, dans lequel, lors de la préparation du mélange des composants (a) à (d), on opère sous une pression d'au moins 60 bar et à une température d'au moins 200°C.

5. Procédé selon la revendication 3, dans lequel, lors de la préparation du mélange des composants (a) à (d), on opère sous une pression d'au moins 80 bar et à une température d'au moins 240°C.

6. Procédé selon la revendication 1 ou 2, dans lequel on prépare le mélange à hydrogéner des composants (a) à (d) en mélangeant un ou plusieurs acides gras en C₈₋₂₄ (b) avec du méthanol (c) et en soumettant ce mélange - éventuellement sous une pression élevée et à haute température - à une estérification au moins partielle dans un réacteur d'estérification qui contient un catalyseur d'estérification, solide et immobilisé.

7. Procédé selon l'une quelconque des revendications 1 à 6, dans lequel on effectue l'hydrogénation en présence d'un catalyseur hétérogène en morceaux à base de chromite-Cu.

8. Procédé selon les revendications 1 à 7, dans lequel on effectue l'hydrogénation dans un réacteur à lit fixe.

9. Procédé selon la revendication 7 ou 8, dans lequel on effectue l'hydrogénation dans un réacteur cylindrique ou à faisceau de tubes, en mode opératoire par ruissellement.
